# EUROPEAN PATENT SPECIFICATION

(11) **EP 2 687 154 B1**
(45) Date of publication and mention of the grant of the patent: **07.08.2019**
(21) Application number: 13173734.8
(22) Date of filing: 26.06.2013
(51) Int. Cl.: A61B 5/024, A61B 5/00, A61B 5/11, A61B 5/1455

(54) **Portable device and method for determining a sleep-related parameter of a user**
Tragbare Vorrichtung und Verfahren zur Bestimmung eines schlafbezogenen Parameters eines Benutzers
Dispositif portable et procédé pour déterminer un paramètre de sommeil d'un utilisateur

(30) Priority: 20.07.2012 EP 12177403
(43) Date of publication of application: 22.01.2014
(73) Proprietor: CSEM Centre Suisse d'Electronique et de Microtechnique SA - Recherche et Développement, 2007 Neuchâtel (CH)
(72) Inventor: Renevey, Philippe, 1005 Lausanne (CH); Lemay, Mathieu, 1025 St-Sulpice (CH); Sola i Caros, Josep, 2035 Corcelles (CH); Muntané Calvo, Enrique, 2000 Neuchâtel (CH)
(74) Representative: P&TS SA (AG, Ltd.)

(56) References cited:
- EP-A1- 1 297 784
- EP-A1- 2 116 183
- WO-A2-2008/029399
- WO-A2-2008/029399
- AT-U1- 10 035
- US-A1- 2005 187 446
- US-A1- 2012 083 705
- JOHN ALLEN: "TOPICAL REVIEW; Photoplethysmography and its application in clinical physiological measurement", PHYSIOLOGICAL MEASUREMENT, INSTITUTE OF PHYSICS PUBLISHING, BRISTOL, GB, vol. 28, no. 3, 20 February 2007 (2007-02-20), pages R1-R39, XP020120750, ISSN: 0967-3334, DOI: 10.1088/0967-3334/28/3/R01

## Description

### Field

The present disclosure relates to a portable device comprising a photoplethysmographic sensor and a motion detecting device for determining heart rate variability of a user. The present disclosure further relates to a method for determining a sleep-related parameter from the determined heart rate variability, from the morphology of the PPG waves and from the motion of the subject.

### Description of related art

Sleep stages are usually classified into four categories, namely WAKE, REM, NREM (I, II, III) and UNCLASSIFIED. These classes are defined by the gold standard for sleep classification, the polysomnography. Polysomnography requires an important and invasive monitoring system that is not suitable for long term analysis of sleep.

US2012083705 discloses the preamble of claim 1. More particularly, US2012083705 discloses a PPG sensor intended to be in contact with a tissue of the user and adapted for providing a PPG signal, a motion detecting device for measuring motion of the device on and with respect to the user body tissue and for providing a motion reference signal representative of said motion; and a processing device configured for using the motion reference signal to determine a sleep-related parameter of the user, wherein the sleep-related parameter comprises, activity of the autonomic nervous system of the user while sleeping, or a sleep stage of the user.

EP2116183 A portable cardiovascular monitoring device comprises a first CV sensor based on a photoplethysmography (PPG) technique for measuring a first CV signal of a user and at least a second CV sensor based on an electrocardiography (ECG) or an impedance cardiography (ICG) method for measuring a second CV signal. The portable cardiovascular monitoring device comprises a control device (13) and a signal processing module (14) for estimating the reliability of the CV sensor signals and selecting at least one CV sensor signals to be utilized in order to obtain a single robust user's heart rate estimation.

### Summary

The present disclosure concerns a portable device for the monitoring of sleep and sleep stages of a user, comprising:
a photoplethysmographic sensor intended to be in contact with a tissue of the user and adapted for delivering cardiovascular pulse wave related PPG signal;
a motion detecting device for measuring motion of the device on and with respect to the user body tissue and for providing a motion reference signal representative of said motion and determining a user activity from the motion reference signal; and
a processing device configured for determining beat-to-beat intervals from the PPG signal;
the processing device being further configured for determining heart rate variability using the determined beat-to-beat intervals and determining a sleep-related parameter from the determined heart rate variability and the user activity, and for using the motion reference signal in combination with said beat-to-beat intervals to determine different sleep stages of the user, wherein the sleep-related parameter comprises, activity of the autonomic nervous system of the user while sleeping, or a sleep stage of the user;
the processing device being further configured for using the motion reference signal to select reliable portions of the PPG signal when the motion reference signal is below a threshold movement value and to reject portions of the PPG when the motion reference signal is equal or above the threshold movement value; and
the processing device being further configured for determining beat-to-beat intervals from a time-series of beat-to-beat intervals and determining the position of the heart beats by searching minimal value of the time-derivative of the selected reliable portions of the PPG signal.

The present disclosure further concerns a method for determining a sleep-related parameter of a user wearing the portable device provided in contact with a tissue of the user, the method comprising:
providing the PPG signal by using the PPG sensor;
providing the motion reference signal by using the motion detecting device;
determining the user activity from the motion reference signal;
determining a reliability of the PPG signal by combining the PPG signal with the motion reference signal;
determining beat-to-beat intervals from the PPG signal and the determined reliability;
determining heart rate variability using the determined beat-to-beat intervals;
determining a sleep-related parameter from the determined heart rate variability and the user activity; and
determining activity of the autonomic nervous system of the user during sleep, or a sleep stage of the user, using said sleep-related parameter;
wherein determining a reliability of the PPG signal comprises using the motion reference signal to select reliable portions of the PPG signal when the motion reference signal is below a threshold movement value and to reject portions of the PPG when the motion reference signal is equal or above the threshold movement value; and
wherein said determining beat-to-beat intervals comprises determining a time-series of beat-to-beat intervals and determining the position of the heart beats by searching minimal value of the time-derivative of the selected reliable portions of the PPG signal.

The sleep-related parameter determined using the method and device described herein can be used for monitor non-invasively sleep quality of the user. The motion reference signal allows for improving the robustness of the determined sleep-related parameter. The portable device can be advantageously used for consumer market, for example in wellness applications, as well as for advanced medical monitoring such as the variation of mood state of bipolar patients or more generally all sleep disorders requiring long-term monitoring.

### Brief Description of the Drawings

The invention will be better understood with the aid of the description of an embodiment given by way of example and illustrated by the figures, in which:
Fig. 1 shows a portable device comprising a photoplethysmographic sensor and a motion detecting device, according to an embodiment.
Fig. 2 illustrates a flow diagram of a method for determining sleep-related parameter of a user using the portable device.

### Detailed Description of possible embodiments

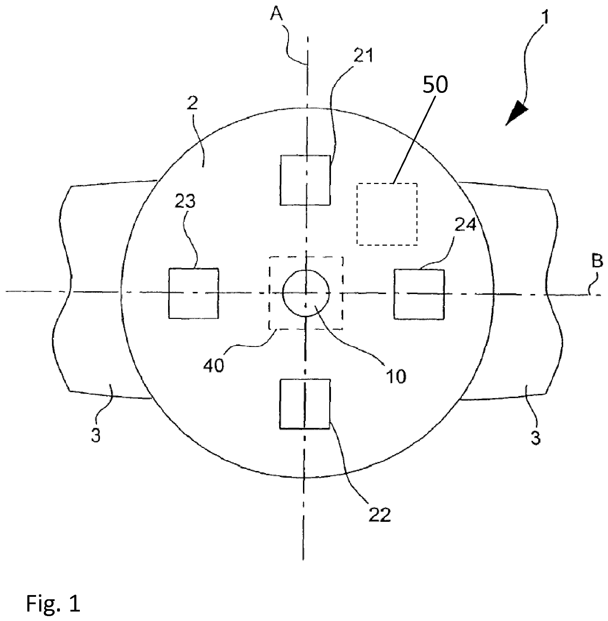

Fig. 1 shows an example of a portable device 1 according to an embodiment. The portable device 1 comprises a light source 10 adapted for emitting a radiant energy signal, or emitted light signal, at the surface of (or through) the human body tissue, and a plurality of light detectors 21 to 24 for measuring the emitted light signal, after propagation through the human body tissue. In the example of Fig. 1, the light source 10 and two pairs of light detectors 21, 22 and 23, 24 are located in a bottom side of a housing 2, in contact with the skin. A strap 3 is used for attaching the housing 2 on the user's wrist.

The light source 10 is preferably an infrared light emitting device (LED). Preferably, the light source 10 emits the emitted light signal with a wavelength that matches the maximum absorption of oxy-haemoglobin. The light detectors may conveniently be photodiodes. The distance between the light source 10 and the light detectors 21, 22 and 23, 24 can be optimized such as to maximize the pulsatile signal that corresponds to the heart beats. In the particular configuration of Fig. 1, the light source 10 is located in a substantially central part of bottom side and the light detectors 21 to 24 are disposed around and at a determined distance from light source 10. In this example, this distance is advantageously selected to be approximately equal to 10 mm.

The light source 10 emits an emitted light signal, preferably an infrared (IR) signal, at the surface of the human body tissue (or alternatively through the body tissue). The emitted light signal propagates through the tissue where it is submitted to modifications due to reflection, refraction, scattering and absorption and is received by the light detectors 21-24. Since variations of optical tissue characteristics are related to variations in the subcutaneous blood flow, the received signal, or PPG signal, can be used for the estimation of the heart rate. In the case of an IR emitted light signal, the absorption is mainly due to chromophores such as haemoglobin, myoglobin, cytochrome, melanin, lipid, bilirubin, and water. The relative importance depends on the wavelength considered and their distribution in the tissue.

It will be appreciated that at least two light detectors are required for a proper detection of the heart rate. The measurement device could thus be designed to have two, three or even more than four light detectors. The number and spatial arrangement of these light detectors should however be selected in an adequate manner to provide sufficient spatial diversity for removing light-related artifacts. In particular, the use of a plurality of light detectors allows for obtaining a spatial diversity that is necessary to obtain at least one measure of quality in an inhomogeneous tissue such as the wrist.

It will also be appreciated that the measurement device 1 could be designed to be worn on parts of the human body other than the wrist, such as a user's finger, nail, ear lobe or any other suitable member or part of the human body. In addition, the same principles could be applied to a measurement device based on the measurement of light transmitted through the body tissue (such as those typically used in pulse oximetry) where the signal to noise ratio is higher. In addition, these principles could be applied for pulse oximetry on the red and IR emitted light signals.

In another embodiment, the portable device 1 further comprises a second light source (not shown in Fig. 1), also preferably a LED arranged to emit a second emitted light signal having a second wavelength that is different than the wavelength of the emitted light signal. The second light source can be located in the vicinity of the light source 10 such that the optical path of the emitted light signal and second emitted light signal through the tissues is the same. The light source 10 can emit a red emitted light signal and the second light source can emit a near infrared second emitted light signal. Absorption characteristics of red and near infrared light are inverted for haemoglobin and oxy-haemoglobin. It is thus possible to derive the proportion of oxy-haemoglobin and therefore the arterial oxygen saturation and/or blood oxygen concentration from knowledge of the absorption characteristics of the arterial blood at these two wavelengths.

In yet another embodiment not represented, the portable device 1 comprises a regulating device adapted for regulating the power of the light source 10. The regulating device allows for varying the respective amplitude of the emitted light signal in accordance with different measurement conditions. Measurement conditions can comprise the optical properties of the user's tissue, illumination properties conditions of the light source, the type and degree of tightening of the portable device, local properties of the user's tissue, etc. For example, the regulating device can be used for adjusting the amplitude of the emitted light signal such as to maximize the amplitude of the emitted light signals without saturating the light detectors 21-24.

In yet another embodiment, the portable device 1 further comprises a motion detecting device 40 which is for example disposed in an upper part of housing 2. This motion detecting device 40 is preferably a three dimensional accelerometer, that is, in effect, three accelerometers disposed along three orthogonal measurement axes and providing three dimensional acceleration data representative of the acceleration to which the device is subjected. It will however be appreciated that other types of accelerometers or motion detecting devices could be used provided they deliver a reliable measure of motion of the pulse rate detecting device on and with respect to the human body tissue.

In yet another embodiment, the portable device 1 further comprises a processing device 50 for the processing of the PPG signals and/or the motion reference signal. The processing device 50 can be located on the portable device 1 as in the example of Fig. 1. The processing device 50 can also be located remote to the portable device 1 and linked to the portable device by means of direct or wireless connection. Preferably, the processing device 50 comprises an adequately programmed digital signal processor or DSP housed within the portable device 1.

Optionally, the portable device 1 may further comprise an outputting device (not shown) for outputting an indication of the processed PPG signals and/or processed motion reference signal. The outputting device can be arranged for outputting an optical, audible signal, or other sensorial signal. For example, the outputting device can comprise a display, a buzzer, a vibrating device or any other suitable device adapted for transmitting information representative of the processed PPG signals and/or the processed motion reference signal to the user.

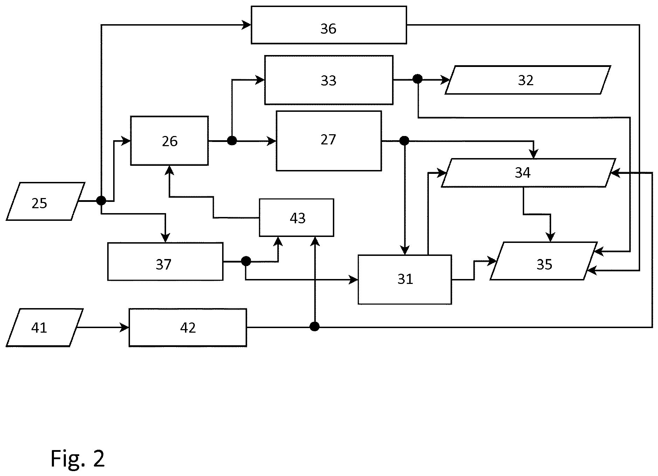

In an embodiment illustrated in the flow diagram of Fig. 2, a method for determining sleep-related parameter of the user using the portable device 1 comprises:
providing the portable device 1 according to any one of claims 1 to 5 in contact with a tissue of the user;
providing the PPG signal (25) by using the PPG sensor
providing the motion reference signal (41) by using the motion detecting device 40;
determining a user activity (42) from the motion reference signal;
determining a reliability (43) of the PPG signal by combining the PPG signal with the motion reference signal;
determining beat-to-beat intervals (26) from the PPG signal and the determined reliability;
determining heart rate variability (HRV) (27) using the determined beat-to-beat intervals; and
determining a sleep-related parameter from the determined HRV and the user activity.

In an embodiment, selecting reliable portions of the PPG signal comprises comparing the motion reference signal with a threshold movement value Th. The reliable portions of the PPG signal are then selected in the case the motion reference signal is below the threshold movement value Th, and are rejected when the motion reference signal is equal or above the threshold movement value Th.

The PPG signal can be compensated for the ambient light by turning the light source 10 off while measuring the emitted light signal with the light detectors 21-24 and processing by a parallel sample-and-hold system (not shown). The measured ambient light can then be subtracted from the PPG signal in order to ensure robustness of the PPG signal to ambient light conditions.

In an embodiment, said determining a time-series of beat-to-beat intervals (26) comprises determining the position of the heart beats by searching minimal value of the time-derivative of the reliable portions of the PPG signal. This can be performed by using an algorithm including physiologically motivated constraints such the refractory period of heart in order to ensure a reliable detection, for example, with inhibition of peak detection during the refractory period of cardiac cells. The sampling frequency of the reliable portions of the PPG signal is a compromise between the autonomy of the portable device 1 and the precision in detecting the heart beat position. A polynomial interpolation of the position of the minima of the time-derivative of the optical measurement is used to improve the precision of the detection without requiring increasing the sampling frequency.

The method can further comprise a step of calculating a reliability index for each beat-to-beat interval of the time-series of beat-to-beat intervals. This can be performed using a nonlinear mapping technique, for example, by using a Multiple Layer Perceptron (MLP), which has been trained on data of various experimental setups.

As discussed above, the reliability index of each beat-to-beat interval can be increased by having the number and spatial arrangement of the light detectors 21-24 such as to provide sufficient spatial diversity for removing motion-related artefacts and other contributions which cannot be detected by the motion detecting device 40. The reliability index of each beat-to-beat interval can further be increased by removing other contributions, such as, for example, reciprocal contributions due to finger movements by using spatio-temporal principal component analysis (PCA).

In yet another embodiment not represented, the PPG sensor can comprise more than two light sources. In this configuration, a PPG signal can be provided for each light source, possibly independently and simultaneously. The emitted light signal for each light source can have different wavelengths. The PPG signals corresponding to the different emitted light signals can then be compared in order to evaluate a similarity score between them. Alternatively, the similarity score can be determined from a PPG parameter calculated from the PPG signal. For example, the PPG parameter can comprise the determined HRV.

The similarity score can then be compared to a threshold similarity score value Ts such as to determine a reliability of the PPG signals. For example, PPG signals having a similarity score being below the threshold similarity score value Ts can have a high reliability index, whereas PPG signals having a similarity score being above the threshold similarity score value Ts can have a low reliability index. In other words, the reliability index depends on the similarity score of the different PPG signal measurements.

Under ideal steady-state condition, the PPG signal contains both a constant component (DC), or continuous component, and a time varying (AC) component, or pulsatile component. The constant component is generally ascribed to baseline absorption of blood and soft tissue, non-expansive tissue such as bone, as well as reflectance loss. The time varying component reflects the modification of the effective path length due to the expansion of the tissues subject to the varying blood pressure.

In an embodiment, the method further comprises extracting the amplitude of the pulsatile component (37) of the PPG signal. In particular, the PPG signal measured by the light detectors 21-24 can be digitalized in the processing device 50 such as to preserve the continuous component of the PPG signal and the pulsatile component of the PPG signal. In an exemplary implementation of the portable device 1, the emitted light signal provided by the light source 10 can be pulsed such as to lower power consumption of the portable device 1 and the processing device 50 can comprise a sample-and-hold electronics used for recovering the continuous component of the PPG signal. Extracting said amplitude can be performed simultaneously with the determination of the beat-to-beat intervals.

In an embodiment, said sleep-related parameter comprises a vascularization parameter. Determining the vascularization parameter can comprise extracting the amplitude of the pulsatile part (AC) of the PPG signal. The amplitude of the pulsatile part (AC) of the PPG signal is a direct measurement of the vascularization of the user's tissues being in contact with the PPG sensor. The variation in amplitude of the pulsatile part during sleep can be used as an indicator of the activity of the autonomic nervous system (ANS) of the user. The method can further comprise estimating the continuous component for each beat-to-beat interval of the beat-to-beat interval time-series, as well as the current provided to the light source 10. The amplitude of the pulsatile part of the PPG signal can then be normalized by using the continuous component and the current.

HRV can be determined from the determined beat-to-beat intervals by using time-domain methods yielding variables such as SDNN (standard deviation of the determined beat-to-beat intervals) or pNN50. The SDNN and pNN50 variables can be computed by using a sliding constant length window.

In an embodiment, determining HRV can comprise resampling the determined beat-to-beat intervals such as to obtain a uniformly resampled beat-to-beat interval time series. The resampling is preferably performed at a frequency of 5Hz. The beat-to-beat interval time series can be weighted using the reliability index.

In an embodiment, said sleep-related parameter comprises a respiration frequency parameter. In order to determine the respiration frequency parameter, the method can further comprise estimating the respiration frequency 31 from the modulation of resampled beat-to-beat interval time series combined with the modulation of the amplitude of the pulsatile component (37) of the PPG signal; and tracking the frequency with maximum power in the HRV spectrum related to said respiration frequency. The tracking can be performed by using an adaptive notch filter.

Extracting the power in different frequency bands can further be related to the autonomic nervous system such as very low frequency (VLF), low frequency (LF) and high frequency (HF). Said extracting the power in different frequency bands can comprise using band-pass filters, estimation of auto-regressive model estimation based on the autocorrelation matrix, NLMS, filter bank, or any other methods suitable for the estimation of power spectrum.

In another embodiment, said sleep-related parameter comprises an oxygenation parameter. For that purpose, the portable device 1 can further comprise the second light source emitting a light signal having a second wavelength that is different than the one of the emitted light signal of the first light source 10 such as to provide a second PPG signal (see above). Determining the oxygenation parameter comprises estimating blood oxygen concentration (36) from the PPG signal and the second PPG signal.

In an embodiment, said sleep-related parameter comprises an arrhythmia parameter, or extrasystole detector, (32). Identification of arrhythmia is based on reliable estimation of abnormal beat-to-beat interval, or abnormal inter-beat patterns (33), which in turn is based on using reliable portions of the PPG signal. The arrhythmia parameter can be based on different type of arrhythmias such as bradycardia that is identified for heart rate being below 60 beats per minute, or sinus / ventricular tachycardia identified for heart rate being above 100 beats per minute. Other possible arrhythmia parameters can include atrial flutter, atrial fibrillation and ectopic beat. Identification of sinus / ventricular tachycardia, atrial flutter and atrial fibrillation should be preferably performed in combination with the motion reference signal such as to rule out high heart rate due to movements of the user.

Abnormal variation of beat-to-beat intervals, combined with and absence of respiratory component can be used to detect apnea (35).

The estimated concentration of oxygen in blood can be used for improving the classification of sleep (34) by detecting possible problems in the ventilation (severe apnea). For example, a decrease in oxygen in blood can be associated with apnea event that degrade the quality of sleep.

Said sleep-related parameter (HRV signals, respiratory signals and activity signals) can be used for determining a sleep stage of the user. This can be performed by using a classification system. The classification system can comprise a hidden Markov model (HMM) or any other suitable machine learning system such as Baysien, Support Vector Machine or Artificial Neural Networks. Initially, the classification system is trained by supervised database where said sleep-related parameters are recorded in parallel with an expert-supervised polysomnography. Once the classification system is able to predict polysomnographic stages such as WAKE, REM, NREM and UNCLASSIFIED, the training phase is finished and the trained parameters are stored in the classification system. For any additional user, the classification system is then able to predict, in an unsupervised fashion, the user's current sleep stage.

As discussed above, said sleep-related parameter can further be used for determining the activity of the autonomic nervous system (ANS) of the user and therefore providing an information about general stress level or night recovery after a strenuous physical activity.

The portable device 1 and the method described herein can be advantageously used in the domains of wellness and sport. Indeed, the portable device 1 is adapted to non-invasively determine the quality of sleep and can provide information about general stress level and quality of life-style. The portable device 1 and method can also be used for the monitoring of the effect of training in sport and detect over-training. The portable device 1 and method can further be used to monitor patient whose pathologies are related to alteration of sleep. This can include, non-exhaustively, mood disorders such as depression or bipolar disorder, alteration of sleep, evaluation of the effects of different medications on sleep quality. The portable device 1 and method allows for implementing long-term research involving study of sleep. The portable device 1 and the associated method are particularly suited for long-term analysis of sleep due to their unobtrusiveness.

### Reference numbers

- 1: portable device
- 2: housing
- 3: strap
- 10: first light source
- 21-24: light detector
- 25: providing PPG signal
- 26: determining beat-to-beat intervals
- 27: determining heart rate variability
- 31: estimating the respiration frequency
- 32: detection of arrhythmia
- 33: detection of abnormal inter-beat patterns
- 34: classification of sleep phase
- 35: detection of apnea
- 36: determining blood oxygen concentration
- 37: extracting amplitude of pulsatile component
- 40: motion detecting device
- 41: providing the motion signal
- 42: determining a user activity
- 43: determining a reliability of PPG signal
- 50: processing device

- Th: threshold movement value
- Ts: threshold similarity score value

## Claims

1. A portable device for the monitoring of sleep of a user, comprising:
a photoplethysmographic (PPG) sensor intended to be in contact with a tissue of the user and adapted for providing a PPG signal;
a motion detecting device (40) for measuring motion of the device (1) on and with respect to the user body tissue and for providing a motion reference signal representative of said motion and determining a user activity (42) from the motion reference signal; and
a processing device (50) configured for determining beat-to-beat intervals from the PPG signal;
the processing device (50) being further configured for determining heart rate variability (27) using the determined beat-to-beat intervals and determining a sleep-related parameter from the determined heart rate variability and the user activity, and for using the motion reference signal in combination with said beat-to-beat intervals to determine a sleep-related parameter of the user, wherein the sleep-related parameter comprises, activity of the autonomic nervous system of the user while sleeping, or a sleep stage of the user;
**characterized in that**
the processing device (50) is further configured for using the motion reference signal to select reliable portions of the PPG signal when the motion reference signal is below a threshold movement value (Th) and to reject portions of the PPG when the motion reference signal is equal or above the threshold movement value (Th); and
**in that** the processing device (50) is further configured for determining beat-to-beat intervals from a time-series of beat-to-beat intervals (26) and determining the position of the heart beats by searching minimal value of the time-derivative of the selected reliable portions of the PPG signal.

2. The device according to claim 1, wherein
said PPG sensor comprises a first light-emitting source (10) for emitting a first light signal having a first wavelength at the surface of or through a user body tissue; and a plurality of light detectors (21-24) for detecting the intensity of said light signal after propagation through the user body tissue such as to provide the first PPG signal.

3. The device according to claim 2,
further comprising a second light-emitting source emitting a second light signal having a second wavelength that is different than the first wavelength, such that a value of oxygen saturation and/or blood oxygen concentration can be determined from the first and second PPG signals.

4. The device according to claim 3,
further comprising a regulating device adapted for regulating the power of the first and second light source (10) such that the amplitude of the first and second light signals can be varied in accordance with measurement conditions.

5. A method for determining a sleep-related parameter of a user comprising:
providing the portable device (1) according to any one of claims 1 to 4 in contact with a tissue of the user;
providing the PPG signal (25) by using the PPG sensor;
providing the motion reference signal (41) by using the motion detecting device (40);
determining a user activity (42) from the motion reference signal;
determining a reliability (43) of the PPG signal by combining the PPG signal with the motion reference signal;
determining beat-to-beat intervals (26) from the PPG signal and the determined reliability;
determining heart rate variability (HRV) (27) using the determined beat-to-beat intervals;
determining a sleep-related parameter from the determined HRV and the user activity; and
determining activity of the autonomic nervous system of the user during sleep, or a sleep stage of the user, using said sleep-related parameter;
**characterized in that**, determining a reliability (43) of the PPG signal comprises using the motion reference signal to select reliable portions of the PPG signal when the motion reference signal is below a threshold movement value (Th) and to reject portions of the PPG when the motion reference signal is equal or above the threshold movement value (Th); and
said determining beat-to-beat intervals comprises determining a time-series of beat-to-beat intervals (26) and determining the position of the heart beats by searching minimal value of the time-derivative of the selected reliable portions of the PPG signal.

6. The method according to claim 5, wherein
said determining a sleep stage (34) comprises using a classification system adapted to predict polysomnographic stages comprising WAKE, REM or NREM.

7. Method according to any one of the claims 5 to 6, wherein the PPG sensor comprises more than two light sources, each light source being adapted to provide a PPG signal; and wherein
the method further comprises providing a plurality of PPG signals;
comparing said plurality of PPG signals in order to provide a similarity score between them;
determining a reliability index from the similarity score.

8. Method according to claim 7, wherein
the similarity score is determined by comparing each of said plurality of PPG signals between each other, or from a PPG parameter calculated from the PPG signals.

9. Method according to claim 8, wherein
said PPG parameter comprises the determined HRV.

10. The method according to any one of the claims 5 to 9, wherein said sleep-related parameter comprises a respiration frequency parameter and wherein
said determining the respiration frequency parameter comprises:
estimating the respiration frequency (31) from the modulation of the resampled beat-to-beat interval time series and from the modulation of the amplitude of the pulsatile component of the PPG signals; and
tracking the frequency with maximum power in the HRV spectrum related to said respiration frequency.

11. The method according to any one of the claims 5 to 10, wherein
said sleep-related parameter comprises a vascularization parameter and wherein
said determining the vascularization parameter comprises extracting the amplitude of the pulsatile part (37) of the PPG signal.

12. The method according to claim 11,
further comprising extracting the continuous component of the PPG signal and a current provided to the light source (10); and
using the continuous component and the current for normalizing the amplitude of the pulsatile part of the PPG signal.

13. The method according to any one of the claims 5 to 12, wherein
said sleep-related parameter comprises an oxygenation parameter and wherein
the portable device (1) further comprises a second light source emitting a light signal having a second wavelength that is different from the one of the emitted light signal of the first light source (10) such as to provide a second PPG signal; and wherein
said determining the oxygenation parameter comprises estimating blood oxygen concentration (36) from the PPG signal and the second PPG signal.

14. The method according to claim 5, wherein
said sleep-related parameter comprises an arrhythmia parameter or extrasystole detector, and wherein
said determining the arrhythmia or extrasystole detector (32) comprises using the determined beat-to-beat intervals and subsequent HRV analysis in combination with the motion by selecting the reliable portions of the PPG signal corresponding to the motion reference signal being below the threshold movement value (Th).

## Patentansprüche

1. Tragbare Vorrichtung zum Überwachen des Schlafes eines Benutzers, umfassend:
einen photoplethysmographischen (PPG) -Sensor, der mit einem Gewebe des Benutzers in Kontakt stehen soll und zum Liefern eines PPG-Signals eingerichtet ist;
eine Bewegungserfassungsvorrichtung (40) zum Messen der Bewegung der Vorrichtung (1) auf und in Bezug auf das Körpergewebe des Benutzers und zum Liefern eines Bewegungsreferenzsignals, welches für die Bewegung repräsentativ ist, und Ermitteln einer Benutzeraktivität (42) aus dem Bewegungsreferenzsignal; und
eine Verarbeitungsvorrichtung (50), welche zum Ermitteln von Schlag-zu-Schlag-Intervallen aus dem PPG-Signal konfiguriert ist;
wobei die Verarbeitungsvorrichtung (50) zudem konfiguriert ist, die Herzfrequenzvariabilität (HRV) (27) unter Verwendung der ermittelten Schlag-zu-Schlag-Intervalle zu bestimmen und einen schlafbezogenen Parameter aus der ermittelten Herzfrequenzvariabilität und der Benutzeraktivität zu ermitteln, und das Bewegungsreferenzsignal in Kombination mit den besagten Schlag-zu-Schlag-Intervallen zu verwenden, um einen schlafbezogenen Parameter des Benutzers zu ermitteln, worin der schlafbezogene Parameter eine Aktivität des autonomen Nervensystems des Benutzers während des Schlafens oder ein Schlafstadium des Benutzers umfasst;
**dadurch gekennzeichnet, dass**
die Verarbeitungsvorrichtung (50) zudem konfiguriert ist, um das Bewegungsreferenzsignal zu verwenden, um zuverlässige Teile des PPG-Signals auszuwählen, wenn das Bewegungsreferenzsignal unter einem Bewegungsschwellenwert (Th) liegt und Teile des PPG zurückzuweisen, wenn das Bewegungsreferenzsignal gleich mit oder über dem Bewegungsschwellenwert (Th) liegt; und
die Verarbeitungsvorrichtung (50) zudem konfiguriert ist, um Schlag-zu-Schlag-Intervalle aus einer Zeitreihe von Schlag-zu-Schlag-Intervallen zu ermitteln, und um die Position der Herzschläge zu ermitteln, indem ein Minimumwert der zeitlichen Ableitung der ausgewählten zuverlässigen Teile des PPG-Signals gesucht wird.

2. Vorrichtung gemäss Anspruch 1, worin der PPG-Sensor eine erste Lichtemissionsquelle (10) zum Emittieren eines ersten Lichtsignals mit einer ersten Wellenlänge an der Oberfläche von oder durch ein Körpergewebe eines Benutzers umfasst; und eine Vielzahl von Lichtdetektoren (21-24) zum Erfassen der Intensität des besagten Lichtsignals nach der Ausbreitung durch das Körpergewebe des Benutzers, um so das erste PPG-Signal zu liefern.

3. Vorrichtung gemäss Anspruch 2, zudem umfassend eine zweite Lichtemissionsquelle, welche ein zweites Lichtsignal mit einer zweiten Wellenlänge emittiert, die sich von der ersten Wellenlänge unterscheidet, so dass ein Wert der Sauerstoffsättigung und/oder Blutsauerstoffkonzentration aus den ersten und zweiten PPG-Signalen bestimmt werden kann.

4. Vorrichtung gemäss Anspruch 3, zudem umfassend eine Regelvorrichtung, die zum Regeln der Leistung der ersten und zweiten Lichtquelle (10) derart eingerichtet ist, dass die Amplitude des ersten und des zweiten Lichtsignals gemäss den Messbedingungen variiert werden kann.

5. Verfahren zum Bestimmen eines schlafbezogenen Parameters eines Benutzers, umfassend:
Bereitstellen der tragbaren Vorrichtung (1) gemäss einem der Ansprüche 1 bis 4 in Kontakt mit einem Gewebe des Benutzers;
Bereitstellen des PPG-Signals (25) unter Verwendung des PPG-Sensors;
Bereitstellen des Bewegungsreferenzsignals (41) unter Verwendung der Bewegungserfassungsvorrichtung (40);
Bestimmen einer Benutzeraktivität (42) aus dem Bewegungsreferenzsignal;
Bestimmen einer Zuverlässigkeit (43) des PPG-Signals durch Kombinieren des PPG-Signals mit dem Bewegungsreferenzsignal;
Bestimmen von Schlag-zu-Schlag-Intervallen (26) aus dem PPG-Signal und der bestimmten Zuverlässigkeit;
Bestimmen der Herzfrequenzvariabilität (HRV) (27) unter Verwendung der ermittelten Schlag-zu-Schlag-Intervalle;
Bestimmen eines schlafbezogenen Parameters aus der ermittelten HRV und der Benutzeraktivität; und
Bestimmen der Aktivität des autonomen Nervensystems des Benutzers während des Schlafens oder eines Schlafstadiums des Benutzers, unter Verwendung des besagten schlafbezogenen Parameters;
**dadurch gekennzeichnet, dass**
das Bestimmen der Zuverlässigkeit (43) des PPG-Signals die Verwendung des Bewegungsreferenzsignals umfasst, um zuverlässige Teile des PPG-Signals auszuwählen, wenn das Bewegungsreferenzsignal unter einem Bewegungsschwellenwert (Th) liegt und Teile des PPG zurückzuweisen, wenn das Bewegungsreferenzsignal gleich mit oder über dem Bewegungsschwellenwert (Th) liegt; und
das besagte Bestimmen der Schlag-zu-Schlag-Intervalle es umfasst, eine Zeitreihe von Schlag-zu-Schlag-Intervallen (26) zu ermitteln und die Position der Herzschläge zu ermitteln, indem ein Minimumwert der zeitlichen Ableitung der ausgewählten zuverlässigen Teile des PPG-Signals gesucht wird.

6. Verfahren gemäss Anspruch 5, worin das Bestimmen einer Schlafstufe (34) die Verwendung eines Klassifizierungssystems umfasst, das zur Vorhersage von polysomnographischen Stufen einschliesslich WAKE, REM oder NREM angepasst ist.

7. Verfahren gemäss irgendeinem der Ansprüche 5 bis 6, worin der PPG-Sensor mehr als zwei Lichtquellen umfasst, wobei jede Lichtquelle dazu eingerichtet ist, ein PPG-Signal bereitzustellen; und worin
das Verfahren zudem das Bereitstellen mehrerer PPG-Signale umfasst;
das Vergleichen der Vielzahl von PPG-Signalen, um eine Ähnlichkeitsbewertung zwischen ihnen bereitzustellen;
das Bestimmen eines Zuverlässigkeitsindex aus der Ähnlichkeitsbewertung.

8. Verfahren gemäss Anspruch 7, worin die Ähnlichkeitsbewertung durch Vergleichen jedes der Vielzahl von PPG-Signalen untereinander oder aus einem aus den PPG-Signalen berechneten PPG-Parameter bestimmt wird.

9. Verfahren gemäss Anspruch 8, worin der PPG-Parameter die bestimmte HRV umfasst.

10. Verfahren gemäss irgendeinem der Ansprüche 5 bis 9, worin der schlafbezogene Parameter einen Beatmungsfrequenzparameter umfasst, und worin das Bestimmen des Beatmungsfrequenzparameters umfasst:
Schätzen der Beatmungsfrequenz (31) aus der Modulation der neu abgetasteten Zeitreihe von Schlag-zu-Schlag-Intervallen und aus der Modulation der Amplitude der pulsierenden Komponente der PPG-Signale; und
Verfolgen der Frequenz mit maximaler Leistung in dem auf die Atmungsfrequenz bezogenen HRV-Spektrum.

11. Verfahren gemäss irgendeinem der Ansprüche 5 bis 10, worin der besagte schlafbezogene Parameter einen Vaskularisierungsparameter umfasst, und
worin das besagte Bestimmen des Vaskularisierungsparameters das Extrahieren der Amplitude des pulsierenden Teils (37) des PPG-Signals umfasst.

12. Verfahren gemäss Anspruch 11, zudem umfassend das Extrahieren der kontinuierlichen Komponente des PPG-Signals und eines Stroms, der an die Lichtquelle (10) geliefert wird; und
Verwenden der kontinuierlichen Komponente und des Stroms zum Normalisieren der Amplitude des pulsierenden Teils des PPG-Signals.

13. Verfahren gemäss irgendeinem der Ansprüche 5 bis 12, worin der besagte schlafbezogene Parameter einen Sauerstoffanreicherungsparameter umfasst, und worin
die tragbare Vorrichtung (1) zudem eine zweite Lichtquelle umfasst, welche ein Lichtsignal emittiert, das eine zweite Wellenlänge aufweist, die sich von derjenigen des emittierten Lichtsignals der ersten Lichtquelle (10) unterscheidet, um ein zweites PPG-Signal zu liefern; und worin
das besagte Bestimmen des Sauerstoffanreicherungsparameters das Schätzen der Blutsauerstoffkonzentration (36) aus dem PPG-Signal und dem zweiten PPG-Signal umfasst.

14. Verfahren gemäss Anspruch 5, worin der besagte schlafbezogene Parameter einen Arrhythmieparameter oder einen Extrasystolendetektor umfasst, und worin
das besagte Bestimmen der Arrhythmie oder des Extrasystolendetektors (32) es umfasst, die ermittelten Schlag-zu-Schlag-Intervalle und die anschliessende HRV-Analyse in Kombination mit der Bewegung zu verwenden, durch Auswählen der zuverlässigen Teile des PPG-Signals, die dem Bewegungsreferenzsignal unterhalb des Bewegungsschwellenwerts (Th) entsprechen.

## Revendications

1. Dispositif portable pour la surveillance du sommeil d'un utilisateur, comprenant:
un capteur photopléthysmographique (PPG) destiné à être en contact avec un tissu de l'utilisateur et adapté pour fournir un signal PPG;
un dispositif de détection de mouvement (40) pour mesurer le mouvement du dispositif (1) sur le et par rapport au tissu corporel de l'utilisateur et pour fournir un signal de référence de mouvement représentatif dudit mouvement et déterminer une activité d'utilisateur (42) à partir du signal de référence de mouvement; et
un dispositif de traitement (50) configuré pour déterminer des intervalles battement par battement à partir du signal PPG ;
le dispositif de traitement (50) étant en outre configuré pour déterminer la variabilité de la fréquence cardiaque (VRC) (27) en utilisant les intervalles battement par battement déterminés et pour déterminer un paramètre lié au sommeil à partir de la variabilité déterminée de la fréquence cardiaque et de l'activité de l'utilisateur, et pour utiliser le signal de référence de mouvement en combinaison avec lesdits intervalles battement par battement pour déterminer un paramètre lié au sommeil de l'utilisateur, dans lequel le paramètre lié au sommeil comprend l'activité du système nerveux autonome de l'utilisateur pendant le sommeil ou une phase de sommeil de l'utilisateur;
**caractérisé en ce que**
le dispositif de traitement (50) est en outre configuré pour utiliser le signal de référence de mouvement pour sélectionner des portions fiables du signal PPG lorsque le signal de référence de mouvement est inférieur à une valeur-seuil de mouvement (Th) et pour rejeter les portions du PPG lorsque le signal de référence de mouvement est égal ou supérieur à la valeur-seuil de mouvement (Th) ; et
**en ce que** le dispositif de traitement (50) est en outre configuré pour déterminer les intervalles battement par battement à partir d'une série temporelle d'intervalles battement par battement (26) et pour déterminer la position des battements cardiaques en cherchant la valeur minimale de la dérivée de temps des portions fiables sélectionnées du signal PPG.

2. Dispositif selon la revendication 1, dans lequel ledit capteur PPG comprend une première source émettrice de lumière (10) pour émettre un premier signal lumineux ayant une première longueur d'onde à la surface de ou au travers d'un tissu corporel de l'utilisateur ; et une pluralité de détecteurs de lumière (21-24) pour détecter l'intensité dudit signal lumineux après propagation à travers le tissu corporel de l'utilisateur de manière à fournir le premier signal PPG.

3. Dispositif selon la revendication 2, comprenant en outre une deuxième source émettrice de lumière émettant un deuxième signal lumineux ayant une deuxième longueur d'onde différente de la première longueur d'onde, de sorte qu'une valeur de saturation en oxygène et/ou de concentration en oxygène dans le sang puisse être déterminée à partir des premiers et deuxièmes signaux PPG.

4. Dispositif selon la revendication 3, comprenant en outre un dispositif de régulation adapté pour réguler la puissance des premières et deuxièmes sources de lumière (10) de telle sorte que l'amplitude des premiers et deuxièmes signaux lumineux puisse être modifiée en fonction des conditions de mesure.

5. Procédé pour déterminer un paramètre lié au sommeil d'un utilisateur comprenant:
de fournir le dispositif portable (1) selon l'une quelconque des revendications 1 à 4 en contact avec un tissu de l'utilisateur;
de fournir le signal PPG (25) en utilisant le capteur PPG;
de fournir le signal de référence de mouvement (41) en utilisant le dispositif de détection de mouvement (40);
de déterminer une activité d'utilisateur (42) à partir du signal de référence de mouvement;
de déterminer une fiabilité (43) du signal PPG en combinant le signal PPG avec le signal de référence de mouvement;
de déterminer des intervalles battement par battement (26) à partir du signal PPG et de la fiabilité déterminée;
de déterminer la variabilité de la fréquence cardiaque (VRC) (27) en utilisant les intervalles battement par battement déterminés;
de déterminer un paramètre lié au sommeil à partir du VRC déterminé et de l'activité de l'utilisateur ; et
de déterminer l'activité du système nerveux autonome de l'utilisateur pendant le sommeil ou une phase de sommeil de l'utilisateur, en utilisant ledit paramètre lié au sommeil ;
**caractérisé en ce que**
la détermination d'une fiabilité (43) du signal PPG comprend d'utiliser le signal de référence de mouvement pour sélectionner des portions fiables du signal PPG lorsque le signal de référence de mouvement est en-dessous d'une valeur-seuil de mouvement (Th) et pour rejeter les portions du PPG lorsque le signal de référence de mouvement est égal ou supérieur à la valeur-seuil de mouvement (Th) ; et
ladite détermination d'intervalles battement par battement comprend de déterminer une série temporelle d'intervalles battement par battement (26) et de déterminer la position des battements cardiaques en cherchant la valeur minimale de la dérivée de temps des portions fiables sélectionnées du signal PPG.

6. Procédé selon la revendication 5, dans lequel ladite détermination d'une phase de sommeil (34) comprend l'utilisation d'un système de classification adapté pour prévoir des phases polysomnographiques comprenant WAKE, REM ou NREM.

7. Procédé selon l'une quelconque des revendications 5 à 6, dans lequel le capteur PPG comprend plus de deux sources de lumière, chaque source de lumière étant adaptée pour fournir un signal PPG; et dans lequel
le procédé comprend en outre de fournir une pluralité de signaux PPG;
de comparer ladite pluralité de signaux PPG afin de fournir un score de similarité entre eux;
de déterminer un indice de fiabilité à partir du score de similarité.

8. Procédé selon la revendication 7, dans lequel le score de similarité est déterminé en comparant chacun de ladite pluralité de signaux PPG entre eux, ou à partir d'un paramètre PPG calculé à partir des signaux PPG.

9. Procédé selon la revendication 8, dans lequel ledit paramètre PPG comprend le VRC déterminé.

10. Procédé selon l'une quelconque des revendications 5 à 9, dans lequel ledit paramètre lié au sommeil comprend un paramètre de fréquence de respiration et dans lequel ladite détermination du paramètre de fréquence de respiration comprend:
d'estimer la fréquence de respiration (31) à partir de la modulation de la série temporelle ré-échantillonnée d'intervalles battement par battement et à partir de la modulation de l'amplitude de la composante pulsatile des signaux PPG; et
de suivre la fréquence avec la puissance maximale dans le spectre de VRC en relation avec ladite fréquence de respiration.

11. Procédé selon l'une quelconque des revendications 5 à 10, dans lequel ledit paramètre lié au sommeil comprend un paramètre de vascularisation et dans lequel ladite détermination du paramètre de vascularisation comprend l'extraction de l'amplitude de la partie pulsatile (37) du signal PPG.

12. Procédé selon la revendication 11, comprenant en outre l'extraction de la composante continue du signal PPG et d'un courant fourni à la source de lumière (10); et
l'utilisation de la composante continue et du courant pour normaliser l'amplitude de la partie pulsatile du signal PPG.

13. Procédé selon l'une quelconque des revendications 5 à 12, dans lequel ledit paramètre lié au sommeil comprend un paramètre d'oxygénation et dans lequel
le dispositif portable (1) comprend en outre une deuxième source de lumière émettant un signal lumineux ayant une deuxième longueur d'onde différente de celle du signal lumineux émis par la première source lumineuse (10) de manière à fournir un deuxième signal PPG; et dans lequel
ladite détermination du paramètre d'oxygénation comprend l'estimation de la concentration en oxygène dans le sang (36) à partir du signal PPG et du deuxième signal PPG.

14. Procédé selon la revendication 5, dans lequel
ledit paramètre lié au sommeil comprend un paramètre d'arythmie ou un détecteur d'extrasystole, et dans lequel
ladite détermination du détecteur d'arythmie ou d'extrasystole (32) comprend l'utilisation des intervalles de battement à battement déterminés et l'analyse ultérieure de VRC en combinaison avec le mouvement en sélectionnant les portions fiables du signal PPG correspondant au fait que le signal de référence de mouvement est inférieur à la valeur-seuil de mouvement (Th).
